(19) Europäisches Patentamt
European Patent Office
Office européen des brevets

(11) Publication number: **0 261 781 A1**

(12) # EUROPEAN PATENT APPLICATION

(21) Application number: 87307153.4

(22) Date of filing: 13.08.87

(51) Int. Cl.⁴: C12P 21/00 , C12N 15/00 , //G01N33/573,G01N33/577

The microorganism(s) has (have) been deposited with Institute for Fermentation, Osaka under number(s) IFO 50140, IFO 50141.

(30) Priority: 15.08.86 JP 191898/86
15.08.86 JP 191899/86

(43) Date of publication of application:
30.03.88 Bulletin 88/13

(84) Designated Contracting States:
DE FR GB IT

(71) Applicant: **UNITIKA LTD.**
**No. 50, Higashihonmachi 1-chome**
**Amagasaki-shi Hyogo(JP)**

(72) Inventor: **Suzuki, Tadao c/o Unitika Ltd.**
**Central Research Institute No. 23, Uji Kozakura**
**Uji-shi Kyoto(JP)**
Inventor: **Era, Mihoko c/o Unitika Ltd.**
**Central Research Institute No. 23, Uji Kozakura**
**Uji-shi Kyoto(JP)**

(74) Representative: **Pearce, Anthony Richmond et al**
**MARKS & CLERK Alpha Tower Suffolk Street Queensway Birmingham B1 1TT(GB)**

(54) **Hybridoma cell lines, monoclonal antibodies, and production thereof.**

(57) Hybridoma cell lines obtained by fusion with sensitized lymphocytes obtained from a creatine kinase-immunized animal and capable of producing monoclonal antibodies which are reactive with creatine kinase and which inhibit creatine kinase M subunit activity; monoclonal antibodies produced by such hybridoma cell lines which are reactive with creatine kinase and inhibit creatine kinase M subunit activity; and a method of producing monoclonal antibodies which are reactive with creatine kinase and which inhibit the creatine kinase M subunit activity, which comprises cultivating a hybridoma cell line as described and recovering from the culture the monoclonal antibody which is reactive with creatine kinase and which inhibits creatine kinase M subunit activity, where the use of these antibodies allows simple and easy, high-sensitivity measurement of the CK-MB isozyme by the immunological inhibition method.

EP 0 261 781 A1

# HYBRIDOMA CELL LINES, MONOCLONAL ANTIBODIES, AND PRODUCTION THEREOF

## FIELD OF THE INVENTION

This invention relates to hybridoma cell lines producing monoclonal antibodies usable in assaying creatine kinase (EC 2.7.3.2; hereinafter referred to as "CK" for short) MB isozyme in body fluids, reactive with CK, and capable of inhibiting the CK-M subunit activity, to such monoclonal antibodies (hereinafter referred to as "anti-CK-M activity inhibiting monoclonal antibodies"), and to a method of production thereof.

## BACKGROUND OF THE INVENTION

CK is an enzyme which catalyzes the reversible reactions shown by the equation

$$CP + ADP \underset{}{\overset{CK}{\rightleftarrows}} C + ATP \quad (1)$$

in either direction wherein CP represents creatine-phosphate; C, creatine; ADP, adenosinediphosphate; and ATP, adenosinetriphosphate.

It is known that CK exits in three isoenzymic forms, namely CK-MM, CK-MB and CK-BB, which differ in the combination of two subunits present. CK-MM occurs mainly in muscles such as skeletal muscle and heart muscle, CK-MB in heart muscle, and CK-BB mainly in organs such as brain and kidney [cf. Watts, D.C. The Enzymes, 8, 383-455 (1973)]. It has already been established that specific measurement of CK-MB can provide a particularly useful marker in the diagnosis of myocardial infarction since CK-MB specifically exists in heart muscle [cf. Lang, H.Ed., Creatine Kinase Isoenzymes-Pathophysiology and Clinical Application, Springer Verlag, Berlin-Heidelberg-New York (1981)].

In the area of clinical laboratory tests, measurement of CK activity is one of the important routine tests for the diagnosis of myopathy, neuropathy, central nervous system disorder, psychopathy, cardiopathy, and so forth. Various methods of CK assay have hitherto been proposed. For measuring the enzymatic activity in the reverse reaction (from right to left in the equation shown above), among others, (1) a method comprising determining inorganic phosphate formed upon hydrolysis of CP, (2) a method comprising converting ADP to the reduced form of $\beta$-nicotinamide adenine dinulceotide (NADH) under the action of pyruvate kinase (hereinafter, "PK") and lactate dehydrogenases (LDH) and measuring the decrement in absorbance, and (3) a method comprising converting ADP to pyruvic acid with PK, reacting the pyruvic acid with 2,4-dinitrophenylhydrazine and determining the resultant hydrazone are known.

For measuring the activity for the reaction in the direction from left to right in the above equation among others, (1) a method comprising reacting the resultant C with a dye, followed by colorimetry or fluorometry, (2) a method comprising using luciferase (cf. Japanese Patent Application (OPI) Nos. 41579/76 (corresponding to U.S. Patent Nos. 4,001,088 and 4,080,265), 120796/80, 26200/81 and 105199/82 (corresponding to U.S. Patent No. 4,286,057) (The term "OPI" as used herein refers to a "published unexamined Japanese patent application".), (3) a method comprising using phosphoglycerate kinase (PGK) and glyceraldehyde-3-phosphate dehydrogenase (GAPDH) (cf. Japanese Patent Publication No. 34119/84 (corresponding to U.S. Patent No. 4,352,881) and Japanese Patent Application (OPI) No. 155000/81), (4) a method comprising using hexokinase (HK) and glucose-6-phosphate dehydrogenase (hereinafter, "G6PDH"), and (5) a method comprising using glucokinase (GlcK) and 6PDH are known.

However, these methods cannot distinguish the three isozymes from one another. Therefore, several methods have been proposed for specifically assaying CK-MB, which is a useful marker in diagnosing myocardial infarction. First of all, (1) a method comprising adding an antibody capable of specifically inhibiting the CK-M activity and measuring only the CK-B activity by any of the methods (1)-(5) mentioned above (cf. Japanese Patent Publication No. 19239/81 and the corresponding U.S. Patent No. 4,067,775 as well as Japanese Patent Publication No. 20274/83 and the corresponding U.S. Patent No. 4,330,620) exists. In this case, CK-MB cannot be distinguished from CK-BB, but the amount of CK-BB in serum can generally be ignored. Furthermore, among others, (2) a method comprising separating the CK-MB isozyme on an ion exchange resin and measuring the CK-MB activity by any of the methods (1)-(5) mentioned above (cf. Japanese patent Application (OPI) Nos. 65096/79 and 163886/79), and (3) a method comprising assaying CK-MB in term of protein quantity by an immunoassay technique, not in terms of enzymatic activity [cf.

Clinical Chemistry, 29, 1232 (1983)] are reported. Among these methods, the immunological inhibition method, namely method (1), is the most useful because the procedure is simple and easy and because comparison with total CK activity is easy. In this method, a polyclonal antibody separated from antiserum obtained by immunization with MM isozyme as the antigen is used as the antibody.

Meanwhile, in 1975, Köhler and Milstein showed that single clone-derived, homogeneous antibodies (monoclonal antibodies) can be produced by fusing imunized mouse spleen lymphocytes with myeloma cells and using the resultant hybrid cells (hybridomas) [Nature , 256, 495 (1975)]. Since then, various hybridomas and monoclonal antibodies have been reported [e.g., Koprowski, Proc. Natl. Acad. Sci. USA, 75, 3938 (1978); Galfre et al., Nature, 266, 550 (1977); Köhler et al., Eur. J. Immunol., 6, 511 (1976)]. Several monoclonal antibodies against CK and assay reagents using them have also been reported [e.g., Morris et al., Biochem. J., 213, 417-425 (1983); Chan et al., Clin. Chem., 31, 465-469 (1985); Vaidya et al., Clin. Chem., 32, 657-663 (1986)]. In the reports hereinabove, there is no description of the inhibition of the enzymatic activity of CK by these monoclonal antibodies, but the description only is that the antibodies are usable in ordinary immunoassays for determining the antigenic enzyme in terms of protein quantity.

## SUMMARY OF THE INVENTION

An object of the invention is to provide anti-CK-M activity inhibiting monoclonal antibodies, which are homogeneous and usable in CK-MB isozyme assay as a clinical laboratory test items, as well as a method of producing the same.

Another object of the invention is to provide hybridoma cell lines capable of producing these monoclonal antibodies.

As a result of intensive investigations in an attempt to achieve the above objects, the present invention has now been completed based on the findings that hybridoma cell lines obtained by immunizing an animal with CK-MM or CK-MB as the antigen and fusing lymphocytes derived from this animal and myeloma cells produce anti-CK-M activity inhibiting monoclonal antibodies in large amounts and that the use of these antibodies allows simple, high-sensitivity assay of the CK-MB isozyme by the immunological inhibition method.

The invention thus provides hybridoma cell lines capable of producing monoclonal antibodies which can be used in assaying the MB isozyme of CK in body fluids, which react with CK and inhibit the CK-M subunit activity, such anti-CK-M activity inhibiting monoclonal antibodies, and a method of producing these anti-CK-M activity inhibiting monoclonal antibodies which comprises culturing a hybridoma cell line capable of producing an anti-CK-M activity inhibiting monoclonal antibody and recovering the thus-produced anti-CK-M activity inhibiting monoclonal antibody from the cell culture.

The hybridoma cell lines according to the invention are capable of producing anti-CK-M activity inhibiting monoclonal antibodies and therefore produce these antibodies in large amounts, with ease and at low cost. Furthermore, the anti-CK-M activity inhibiting monoclonal antibodies according to the present invention can be obtained in large amounts, with ease and at low cost, are homogeneous, and satisfactorily inhibit the CK-M subunit activity. Therefore, CK-MB isozyme can be assayed in a simple and easy manner and with good sensitivity by using said antibodies in accordance with the immunological inhibition method as described, for example, in U.S. Patent Nos. 4,067,775 and 4,330,620.

## DETAILED DESCRIPTION OF THE INVENTION

For obtaining monoclonal antibodies according to the invention, a hybridoma cell line capable of producing an anti-CK-M activity inhibiting monoclonal antibody is first prepared. For example, cell lines showing the following cytological properties are suitable as such hybridoma cell lines:

(1) Origin

Fused cells created by fusion between anti-CK-M antibody-producing lymphocytes and myeloma cells.

(2) Morphology

Almost the same in morphology as myeloma cells; for instance, 10-20 μm in size.

(3) Function

Constant production of anti-CK-M activity inhibiting monoclonal antibodies which recognize a single antigenic determinant.

(4) Proliferation rate

Almost the same as the proliferation or multiplication rate for myeloma cells, namely about 10-fold multiplication in 72 hours.

(5) Storability

Very easily storable at -70°C or below for a prolonged period of time.

(6) Optimum proliferation conditions

Temperature 37°C, pH 7.2.

(7) Proliferation ranges

Capable of proliferating within the temperature range of 32-42°C and within the pH range of 6.5-7.8.

Such cell lines may be obtained, for example, in the following manner.

In the first place, the antigen CK-MM or CK-MB is prepared. Thus, CK-MM and CK-MB are separated from each other on DEAE-Sephacell by the method described in Clinica Chimica Acta, 123, 59-71 (1982), for instance. Purified CK-MM, which is usable as the antigen, can be obtained by chromatography of CK-MM on CM-cellulose, Phenyl-Sepharose and/or Butyl-Sepharose.

Purified CK-MB usable as the antigen can be obtained by chromatography on Phenyl-Sepharose of CK-MB separated on DEAE-Sephacell.

Any species of CK-MM or CK-MB can be used as the antigen. From the clinical chemistry viewpoint, however, those species derived from mammals (e.g., human, monkey, swine, cattle, etc.) form which conventional antisera capable of cross reacting with human CK can be obtained and which are different from the animal to be immunized are preferable. These antigens are used to immunize mammals, preferably mice or rats, therewith. The immunization may be performed in a conventional manner. Thus, the dose of antigen, site of administration, use of an adjuvant, and other conditions are substantially the same as in the conventional processes for producing antisera. When mice are used, for instance, each mouse is first given a dose of 0.001 to 10 mg, preferably 0.01 to 1 mg, of CK-MM or CK-MB, together with an adjuvant (e.g., Freund's complete adjuvant) thoroughly admixed therewith, by the subcutaneous, intraperitoneal, or another appropriate route. After three week or more, each mouse is again given such a dose of CK-MM or CK-MB, together with an adjuvant (e.g., Freund's incomplete adjuvant) thoroughly admixed therewith, subcutaneously, intraperitoneally, or by another route. After a further two weeks or more, CK-MM or CK-MB alone is administered to each mouse intravenously, subcutaneously, intraperitoneally, or by another appropriate route at the dose mentioned above for sufficient immunization. The thus-immunized animals are sacrificed preferably 2-4 days after the last immunization, and lymphocytes are collected. For the lymphocyte preparation, the spleen, lymph node, and peripheral blood, among others, are used. the lymphocytes are suspended in a culture medium in a disjoined state.

Separately, myeloma cells are prepared. It is preferable that the myeloma cells be derived from the same species as that of the immunized animals. Furthermore, it is preferable that the myeloma cell line be a drug-resistant mutant strain, most preferably a strain such that unfused myeloma cells cannot grow in a hybridoma selection medium. Most generally, an 8-azaguanine-resistant cell line is used. Such cell line is

deficient in hypoxanthine-guanine phosphoribosyl-transferase and cannot grow in hypoxanthine-aminopterine-thymidine (HAT) medium, one of the selective media which can be used. It is also desirable that the myeloma cells used be incapable of antibody secretion by themselves. For the above reasons, such commercially available cell lines as mouse myeloma P3•X63•Ag8•6•5•3 (X63•6•5•3) or P3•X63•Ag8•U1 (P3U1) and rat myeloma 210•RCY3•Agl•2•3 (each available as Catalogue Nos. 05-565, 09-1597 and 09-1631 from Flow Labolatories, Inc.; an agency in Japan: Dainippon Pharmaceutical Co., Ltd.) are preferably used.

The myeloma cells are cultivated in a medium, such as Eagle's minimal medium (MEM) or RPMI 1640 medium, containing serum, preferably fetal calf serum.

Then, the lymphocytes and myeloma cells obtained in the above manner are suspended in a medium such as MEM or RPMI 1640 for admixture. Although the mixing ratio to be employed on that occasion is optional, it is preferable to use a lymphocyte:myeloma cell ratio by number within the range of 1:1 to 20:1, more preferably within the range of 5:1 to 10:1. The fusion of the mixed cells is effected by using a fusion promoter. The fusion may be performed as described in Immunological Methods, Vol. II, page 285, Academic Press, 1981. Various macromolecular substances, viruses, and so forth can be used as the fusion promoter. The use of polyethylene glycol (PEG) or Sendai virus is preferred, however. The PEG may have an average molecular weight of 400-20,000, but the use of PEG having an average molecular weight of 1,000-7,500 is preferred. PEG is used in a concentration of 40-60 volume percent in the fusion medium.

The fusion promoter is removed from the fused cells by washing and then the fused cells are suspended in MEM or RPMI 1640 supplemented with 5-15 volume percent of serum. The suspension is distributed into wells of a 96-well culture plate or the like in a proportion of 0.5-5 x $10^6$ cells per well. A selective medium (e.g. HAT medium) is then added to each well, followed by medium exchange at appropriate intervals. After 10-14 days, unfused myeloma cells have died and hybridomas alone are found alive and growing. Further, lymphocytes do not live long in vitro but are have died as well in 10-14 days.

Antibody-producing hybridomas can be detected by assaying the culture supernatants for their inhibitory activity. For instance, the supernatants are added to the first reagent of a reagent system for CK activity measurement (as described in Example 4 hereinafter) and the mixtures are used in measuring a certain known level of CK-MM activity. The strain in a well should be selected if the supernatant from this well gives a lower activity value as compared with the activity found for the control (without addition of the supernatant).

In the above manner, hybridoma cell lines capable of producing anti-CK-M activity inhibiting monoclonal antibodies can be produced.

One of the hybridoma cell lines created in the above manner by fusing spleen lymphocytes derived from mice immunized in advance with CK-MM with mouse myeloma cells X63•6•5•3) was named hybridoma CKH-1. This cell line has been deposited with the Institute for Fermentation, Osaka, Japan on January 23, 1986 under the deposit number IFO-50088. When stored in the frozen state at -120°C or below, this CKH-1 cell line can be stored almost indefinitely.

Further, the other typical examples of the hybridoma cell lines used in the present invention include hybridoma CKH-2 and hybridoma CKH-5 which each has been deposited with the Institute for Fermentation, Osaka, Japan on August 11 , 1987 under the deposit numbers IFO-50140 and IFO-50141 , etc.

CKH-1 hybridomas can be cultivated in known media in general use. For instance, they can grow and proliferate in a satisfactory manner in RPMI 1640 or MEM, supplemented with 5-20% of fetal calf serum, at 37°C and in air containing carbon dioxide at a concentration of 5 volume percent. They also have the tumorigenicity of myeloma cells, so that they an proliferate in vivo (e.g., in animals of the same strain or species, or in athymic or nude mice) to produce anti-CK-M activity inhibiting monoclonal antibodies.

Thus, the anti-CK-M activity inhibiting monoclonal antibodies can be obtained in large quantities by cultivating these hybridoma cell lines in vitro or in vivo, as described, for example, in Oi, V.T. and Herzenberg, L.A., Selected Methods in Cellular Immunology, pp 351-372, Freeman and Co. (1980), and Gooding, J.W., Monodonal Antibodies: Principles and Practice, 2nd ed., Academic Press (1986). Methods of collecting the monoclonal antibodies may be classified roughly into two classed. In one approach, the cultivation is performed in a culture vessel., such as a flask, using a medium, and antibody is recovered from the culture supernatant. For example, 0.5-5 x 105 hybridoma cells are inoculated into MEM, or RPMI 1640 medium, containing 5-10 volume percent of serum. In 2-4 days, the degree of multiplication is 10-20 times. Then, antibody is recovered from the supernatant.

In the other approach, the hybridomas thus grown in a culture vessel are inoculated into animals of the same strain or species. Thus, $10^5$-$10^7$ hybridomas are administered to such animals subcutaneously, intraperitoneally or using another appropriate route. After 7-20 days, when hybridoma growth has resulted in large tumor formation, the serum and ascitic fluid are collected. In the case of intraperitoneal administration, the ascitic fluid can be obtained in larger quantities when a mineral oil such as 2,6,10,14-tetramethylpentadecane is administered in advance (3-7 days earlier).

The thus-obtained antibodies can be purified prior to use, as necessary, by using appropriate means applicable to proteins in general, such as fractionation with ammonium sulfate, use of an ion exchanger, or affinity chromatography with CK-M immobilized on the column, as described, for example, in Oi, V.T. and Herzenberg, L.A., Selected Methods in Cellular Immunology, pp. 351-372, Freeman and Co. (9180), and Gooding, J.W., Monodonal Antibodies: Principles and Practice, 2nd ed., Academic Press (1986).

The anti-CK-M activity inhibiting monoclonal antibodies according to the invention have the following physico-chemical properties:

(1) Action

They act on CK to induce an antigen-antibody reaction and thus inhibit the enzymatic activity of the CK-M subunit.

(2) Specificity of antigen-antibody reaction

They react with the CK subunit.

(3) Optimum pH

6-9.

(4) pH range within which they are stable

3-11.

(5) Potency assaying method

The potency is measured in term of the concentration of antibody providing the inhibition ratio of 50% against CK-MM activity as described in Example 1.

(6) Temperature range optimal for their action

20-40°C.

(7) Deactivation conditions

They are deactivated by heating at 100°C for 10 minutes.

(8) Molecular weight

140,000-180,000.

The typical examples of monoclonal antibodies according to the invention includes CKA-1, CKA-2 or CKA-5 as described hereinafter.

The following non-limiting examples are given as further illustrative of the present invention.

Reference Example 1

In the first place, a crude enzyme fraction was prepared by mincing swine heart, followed by homogenization, centrifugation at 30,000 x g and precipitation with 70% ethanol, as described in Clinica Chimica Acta, 123, 59-71 (1982).

Then, the crude enzyme fraction was dissolved and dialized and separated on a DEAE-Sephacell column (pH 7.5) into CK-MM, which flows through the column, and CK-MB, which is adsorbed on the column. The CK-MB fraction was eluted by increasing the sodium chloride concentration. Each fraction was dialyzed against Tris buffer containing 20% of ammonium sulfate, allowed to be adsorbed on a Phenyl-Sepharose column, and eluted by decreasing the ammonium sulphate concentration. The above procedure gave the purified antigens CK-MM and CK-MB.

Example 1

A 50-μg portion of the swine CK-MM obtained in Reference Example 1 was mixed and emulsified in complete Freund's adjuvant (purchased from Nakarai Chemicals) in a mixing ratio of 1:1, and the emulsion was intraperitoneally administered to an 8-week-old Balb/c mouse (purchased from CLEA Japan). Three weeks later, an additional immunization was performed by intravenous administration of 50 μg of the swine CK-MM. Three days thereafter, the spleen was excised, disjoined and suspended in MEM medium (purchased from Nakarai Chemicals) and washed with MEM medium. Separately, mouse myelomas of the cell line X63•6•5•3 (obtained from Kyoto University) were cultivated for 2 days, and cells at the logarithmic growth phase were collected by centrifugation. $10^8$ spleen cells were mixed with $10^7$ myelomas of the cell line X63•6•5•3. Centrifugation gave a pellet, to which 1 ml of 50% PEG 4,000-RPMI 1640 (purchased from Gibco) was gradually added over a 1 minute period on a water bath maintained at 37°C. Then, the mixture was stirred gently for 1 minute and, thereafter, 9ml of RPMI 1640 medium was added gradually to thereby dilute the PEG 4,000. The PEG solution was removed by centrifugation. To the resultant pellet was added 10 ml of HAT medium containing 10% of fetal calf serum. The suspension thus obtained was distributed, in 0.1-ml portions, into the wells of a 96-well culture plate (purchased from Nunc). On the 4th, 8th and 11th day (in total 3 times), half of the culture medium in each well was discarded and a fresh portion of HAT medium was added in its place. On the 14th day, hybridoma growth was observed in 56 of the 96 wells. The supernatant from each of the 56 wells was added to the first reagent of a CK activity measuring reagent system (as described in Example 4) and the resultant mixture was used in assaying a certain known level of CK-MM activity. The strains in those wells which gave lower activity values as compared with the activity found in control assays where no supernatant was added were selected and cloned by the limiting dilution technique. A monoclone, hybridoma CKH-1 (IFO-50088), was thus obtained. The cytological properties of this CKH-1 were in good agreement with those mentioned hereinabove.

Example 2

A 50-μg portion of swine CK-MB obtained in Reference Example 1 was mixed with and emulsified in complete Freund's adjuvant (purchased from Nakarai Chemicals) in a mixing ratio of 1:1, and the emulsion was intraperitoneally administered to an 8-week-old Balb/c mouse (purchased from CLEA Japan). Three weeks later, an additional immunization was performed by intravenous administration of 50 μg of the swine CK-MB. Three days thereafter, the spleen was excised, disjoined and suspended in MEM medium (purchased form Nakarai Chemicals) and washed with MEM medium. The subsequent hybridoma production, screening and cloning, which were performed as described in Example 1, gave a monoclone, hybridoma CKH-2 (IFO-50140 ). The cytological properties of hybridoma CKH-2 were in good agreement with those mentioned hereinabove.

Example 3

A 50-μg portion of swine CK-MM obtained in Reference Example 1 was mixed with and emulsified in complete Freund's adjuvant (purchased from Nakarai Chemicals) in a mixing ratio of 1:1, and the emulsion was administered to an 8-week-old Balb/c mouse (purchased from CLEA Japan). Two weeks later, 50 μg of the swine CK-MM was mixed with and emulsified in incomplete Freund's adjuvant (purchased from Nakarai

Chemicals) in a mixing ratio of 1:1, and the emulsion was subcutaneously administered to the mouse. After a further 3 weeks, 50 $\mu$g of the swine CK-MM was subcutaneously administered for additional immunization. Three days thereafter, the spleen was excised, disjoined and suspended in MEM medium (purchased from Nakarai Chemicals) and washed with MEM medium. The subsequent hybridoma production, screening and cloning, which were performed as described in Example 1, gave a monoclone, hybridoma CKH-5. The cytological properties of this CKH-5 (IFO-50141,) were in good agreement with those mentioned hereinabove.

Example 4

The hybridoma cell line CKH-1 obtained in Example 1 was cultivated in RPMI 1640 medium containing 10% of fetal calf serum. When the cell concentration was 2 x $10^6$ cells/ml, 300 ml of the culture was centrifuged. The supernatant thus collected was fractionated with 50% saturated ammonium sulfate. The crude antibody fraction thus separated was dialyzed, then allowed to be adsorbed on Protein A Sepharose (pH 8.0), and eluted with citrate buffer (pH 4.0) to give 3.2 mg of an anti-CK-M activity inhibiting monoclonal antibody. This antibody was designated CKA-1. It was added to the reagent system for Ck activity measurement as mentioned below and measured for its inhibitory activity against CK-MM activity.

First, a first reagent was prepared, which was composed of 1.4 units/ml of Bacillus stearothermophilus-derived glucokinase (commercially available from Seikagaku Kogyo), 1.2 units/ml of Leuconostoc mesenteroides-derived glucose-6-phosphate dehydrogenase (purchased from Oriental Yeast), 1.2 mM ADP, 0.75 mM NADP, 25 mM glucose, 6.25 mM AMP, 12.4 $\mu$M Ap5A, 12.5 mM N-acetylcysteine, 12.5 mM magnesium acetate, 10 mM sodium azide, 2.5 mM EDTA and 150 mM imidazole acetate buffer (pH 6.7). Then, a second reagent composed of 100 mM CP, 10 mM sodium azide and 25 mM Tris acetate buffer (pH 8.5) was prepared.

Both reagents were allowed to stand in a constant-temperature bath maintained at 30°C. Just prior to their use in CK activity measurement, the first reagent and the second reagent were mixed in a ratio of 4:1.

Thus, 0.5 mg of the anti-CM-M activity inhibiting monoclonal antibody CKA-1 was added to the first reagent mentioned above. To 0.5 ml of the resultant mixture was added CK-MM, CK-MB or CK-BB. Each mixture thus obtained was placed in a cell with a light path length of 1 cm and incubated for 15 minutes. Then, 0.125 ml of the second reagent was added, and the residual CK activity was determined based on the change in absorbance at 340 nm as measured on a spectrophotometer where the cell chamber was maintained at the same constant temperature of 30°C. As controls, the same amount of CK-MM, CK-MB or CK-BB was added to the antibody-free, first reagent and, thereafter, the activity measurement was made in the same manner.

The results thus obtained are shown in Table 1 below.

Table 1

Inhibitory Activity of CKA-1

| Isozyme | Activity Without Addition of Antibody (units/l) | Activity with Addition of Antibody (units/l) | % Inhibition |
|---|---|---|---|
| CK-MM | 210 | 48 | 77 |
| CK-MB | 204 | 124 | 39 |
| CK-BB | 196 | 194 | 1 |

Note: Each value is the mean for three samples.

As shown by the results in Table 1 above, CKA-1 was an antibody capable of inhibiting the CK-M subunit quite well but inhibiting the B subunit very little. Its physico-chemical properties were in good agreement with those mentioned above.

Example 5

The hybridoma cell line CKH-2 obtained in Example 2 was cultivated in the same manner as described in Example 4. from 500 ml of the supernatant, there was obtained 7.8 mg of an anti-CK-M activity inhibiting monoclonal antibody. This antibody was designated CKA-2. It was added to the above-mentioned reagent system for CK activity measurement and the percent inhibition of each CK activity was determined, as described in Example 4.

The results obtained are shown in Table 2 below.

Table 2

Inhibitory Activity of CKA-2

| Isozyme | Activity Without Addition of Antibody (units/l) | Activity with Addition of Antibody (units/l) | % Inhibition |
|---|---|---|---|
| CK-MM | 181 | 33 | 82 |
| CK-MB | 201 | 125 | 62 |
| CK-BB | 196 | 193 | 2 |

Note: Each value is the mean for three samples.

As shown by the results in Table 2, CKA-2 was an antibody capable of inhibiting the CK-M subunit well but inhibiting the B subunit very little. Its physico-chemical properties were in good agreement with those mentioned hereinabove.

Example 6

The hybridoma cell line CKH-5 obtained in Example 3 was cultivated in the same manner as described in Example 4. From 500 ml of the supernatant, 9.5 mg of an anti-CK-M activity inhibiting monoclonal antibody was obtained. This antibody was designated CKA-5. As in Example 4, it was added to the above-mentioned reagent system for CK activity measurement, and the percent inhibition of each CK activity was determined.

The results obtained are shown in Table 3 below.

Table 3

Inhibitory Activity of CKA-5

| Isozyme | Activity Without Addition of Antibody (units/l) | Activity with Addition of Antibody (units/l) | % Inhibition |
|---|---|---|---|
| CK-MM | 476 | 1 | 99.8 |
| CK-MB | 204 | 101 | 50 |
| CK-BB | 198 | 197 | 1 |

Note: Each value is the mean for three samples.

As shown by the results in Table 3, CKA-5 was an antibody capable of inhibiting the CK-M subunit quite but inhibiting the B subunit very little. Its physico-chemical properties were in good agreement with those mentioned hereinabove.

While the invention has been described in detail and with reference to specific embodiments thereof, it will be apparent to one skilled in the art that various changes and modifications can be made therein without departing from the spirit and scope thereof.

## Claims

1. A hybridoma cell line comprising the fusion product of a sensitized lymphocyte obtained from a creatine kinase-immunized animal, and a myeloma cell, said cell line being capable of producing a monoclonal antibody which is reactive with creatine kinase and which inhibits creatine kinase m subunit activity.

2. A hybridoma cell line as claimed in Claim 1, which is capable of producing a monoclonal antibody having the properties of CKA-1, CKA-2 or CKA-5.

3. The hybridoma cell line as claimed in Claim 1 or 2, wherein the hybridoma cell line is CKH-1, CKH-2 or CKH-5.

4. A monoclonal antibody which is reactive with creatine kinase and which inhibits creatine kinase M subunit activity.

5. A monoclonal antibody as claimed in Claim 4, which has the properties of CKA-1, CKA-2 or CKA-5.

6. The monoclonal antibody as claimed in Claim 4 or 5 wherein the monoclonal antibody is CKA-1, CKA-2 or CKA-5.

7. A method of producing a monoclonal antibody which is reactive with creatine kinase and which inhibits creatine kinase M subunit activity comprising
cultivating a hybridoma cell line according to Claim 1, and
recovering from the culture the monoclonal antibody which is reactive with creatine kinase and which inhibits creatine kinase M subunit activity.

European Patent Office

# EUROPEAN SEARCH REPORT

Application number

EP 87 30 7153

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| P,X | CLINICAL CHEMISTRY, vol. 33, no. 6, June 1987, pages 986-987, abstract no. 519, American Association for Clinical Chemistry, US; J.L. DAISS: "Monoclonal antibodies specific for the CK-M subunit and the CKMM dimer" --- | 1-7 | C 12 P 21/00<br>C 12 N 15/00 //<br>G 01 N 33/573<br>G 01 N 33/577 |
| P,X | GENE, vol. 51, 1987, pages 13-19, Elsevier Science Publishers B.V. (Biomedical Division), Amsterdam, NL; P. BUCKEL et al.: "Cloning and nucleotide sequence of heavy- and light-chain cDNAs from a creatine-kinase-specific monoclonal antibody"<br>* Pages 13-14, "Summary", "Introduction" * --- | 1-7 | |
| | | | TECHNICAL FIELDS SEARCHED (Int. Cl.4) |
| X | CHEMICAL ABSTRACTS, vol. 95, no. 15, 12th October 1981, page 234, abstract no. 128032r, Columbus, Ohio, US; U. WUERZBURG et al.: "Immunoinhibition test for creatine kinase-B subunit: a comparison of monoclonal mouse and polyclonal goat antibodies", & J. CLIN. CHEM. CLIN. BIOCHEM. 1981, 19(8), 543-4 ----- | 1-7 | C 12 P<br>C 12 N<br>G 01 N |

The present search report has been drawn up for all claims

| Place of search | Date of completion of the search | Examiner |
| --- | --- | --- |
| THE HAGUE | 05-10-1987 | TURMO Y BLANCO C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document